(19) Europäisches Patentamt
European Patent Office
Office européen des brevets

(11) Veröffentlichungsnummer: **0 258 729**
**B1**

# (12) EUROPÄISCHE PATENTSCHRIFT

(45) Veröffentlichungstag der Patentschrift: **25.04.90**

(21) Anmeldenummer: **87111924.4**

(22) Anmeldetag: **18.08.87**

(51) Int. Cl.⁴: **C07D 211/90**, C07D 491/048, A61K 31/445 // (C07D491/08, 307:00, 211:00)

## (54) Dihydropyridinverbindungen, Verfahren zu ihrer Herstellung und ihre Verwendung.

(30) Priorität: **30.08.86 DE 3629545**

(43) Veröffentlichungstag der Anmeldung:
**09.03.88 Patentblatt 88/10**

(45) Bekanntmachung des Hinweises auf die Patenterteilung:
**25.04.90 Patentblatt 90/17**

(84) Benannte Vertragsstaaten:
**AT BE CH DE ES FR GB GR IT LI NL SE**

(56) Entgegenhaltungen:
**EP-A- 0 125 803**
**EP-A- 0 158 138**
**EP-A- 0 161 221**
**DE-A- 3 445 356**
**FR-A- 2 201 095**
**US-A- 3 970 662**

**Die Akte enthält technische Angaben, die nach dem Eingang der Anmeldung eingereicht wurden und die nicht in dieser Patentschrift enthalten sind.**

(73) Patentinhaber: **BAYER AG, D-5090 Leverkusen 1 Bayerwerk(DE)**

(72) Erfinder: **Goldmann, Siegfried, Dr., Am Osterholz 91, D-5600 Wuppertal 11(DE)**
Erfinder: **Ahr, Hans-Jürgen, Dr.Dr., Claudiusweg 3, D-5600 Wuppertal 1(DE)**
Erfinder: **Puls, Walter, Dr., In den Birken 75, D-5600 Wuppertal 1(DE)**
Erfinder: **Bischoff, Hilmar, Dr., Wilkhausstrasse 107, D-5600 Wuppertal 2(DE)**
Erfinder: **Petzinna, Dieter, Dr.Dr., Peter-Berten-Strasse 10, D-4000 Düsseldorf 12(DE)**
Erfinder: **Schlossmann, Klaus, Dr., In der Beek 116, D-5600 Wuppertal 1(DE)**
Erfinder: **Bender, Joachim, Dr., Steinberger Weg 52, D-5600 Wuppertal 11(DE)**

**EP 0 258 729 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Dihydropyridinverbindungen, Verfahren zu ihrer Herstellung sowie ihre Verwendung in Arzneimitteln, insbesondere von blutzuckerbeeinflussenden Arzneimitteln.

In EP-A 0 158 138 werden bereits Dihydropyridinderivate beschrieben, die sich jedoch von den erfindungsgemäßen Verbindungen strukturell und in ihrer Wirkungsstärke eindeutig unterscheiden. Im Vergleich zu den bekannten Dihydropyridinderivaten zeigen die erfindungsgemäßen Verbindungen insbesondere eine wesentlich stärkere Blutzuckersenkung.

Die vorliegende Erfindung betrifft Dihydropyridinverbindungen der allgemeinen Formel (I)

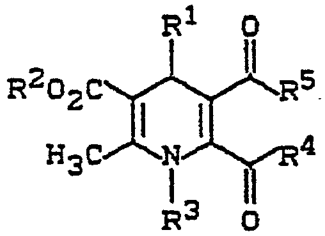

(I)

in welcher

$R^1$ für Phenyl oder Thienyl steht, wobei die genannten Reste bis zu 2 gleiche oder verschiedene Substituenten der Reihe, Fluor, Chlor, Alkyl, Alkoxy mit jeweils bis zu 3 Kohlenstoffatomen, Trifluormethyl, Nitro oder Cyano tragen können,

$R^2$ für geradkettiges oder verzweigtes Alkyl mit bis zu 6 Kohlenstoffatomen steht, das durch ein Sauerstoffatom in der Alkylkette unterbrochen sein kann,

$R^3$ für geradkettiges oder verzweigtes Alkyl mit bis zu 6 Kohlenstoffatomen steht, das durch Hydroxy substituiert sein kann und

$R^4$ und $R^5$ jeweils für Hydroxy stehen, oder

$R^4$ und $R^5$ zusammen für -O- stehen,

in Form ihrer Isomeren, Isomerengemische, optischen Antipoden oder Racemate sowie ihrer physiologisch unbedenklichen Salze.

Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraW Hill, 1962).

Die Dihydropyridindicarbonsäureanhydride im Rahmen der allgemeinen Formel (I) entsprechen der Formel (Ia)

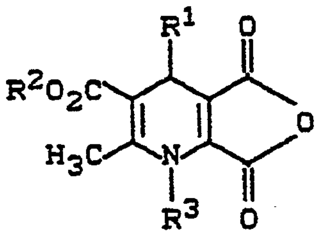

(Ia) ,

die Dihydropyridindicarbonsäuren im Rahmen de allgemeinen Formel (I) entsprechen der Formel (Ib)

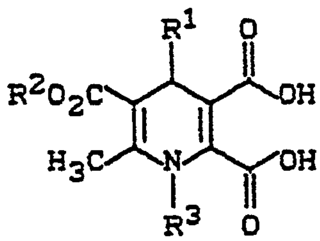

(Ib) .

Die erfindungsgemäßen Verbindungen können in Form ihrer Salze vorliegen. Physiologisch unbedenkliche Salze der Dihydropyridindicarbonsäureanhydride sind im allgemeinen Salze der erfindungsge-

mäßen Stoffe (Ia) mit anorganischen oder organischen Säuren. Als Beispiele seien genannt: Hydrohalogenide, Hydrogensulfate, Sulfate, Hydrogenphosphate, Acetate, Maleate, Citrate, Fumarate, Tartrate, Lactate oder Benzoate.

Physiologisch unbedenkliche Salze der Dihydropyridindicarbonsäuren (Ib) können Metall- oder Ammoniumsalze der erfindungsgemäßen Stoffe sein. Besonders bevorzugt sind z.B. Natrium-, Kalium-, Magnesium- oder Calciumsalze, sowie Ammoniumsalze, die abgeleitet sind von Ammoniak oder organischen Aminen, wie beispielsweise Ethylamin, Di-bzw. Triethylamin, Di-bzw. Triethanolamin, Dicyclohexylamin, Dimethylaminoethanol, Arginin oder Ethylendiamin.

Die erfindungsgemäßen Dihydropyridindicarbonsäureanhydride der allgemeinen Formel (Ia) können hergestellt werden, indem man

Dihydropyridinlactole der allgemienen Formel (II)

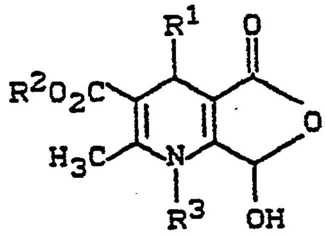
(II)

in welcher $R^1$, $R^2$ und $R^3$ die angegebene Bedeutung haben, in inerten Lösemitteln oxidiert.

Verwendet man als Ausgangsstoff 4-(2-Chlorphenyl)-1-ethyl-7-hydroxy-2-methyl-5-oxo-1,4,5,7-tetrahydrofuro-[3,4-b]pyridin-3-carbonsäureethylester läßt sich die Reaktion durch folgendes Schema verdeutlichen:

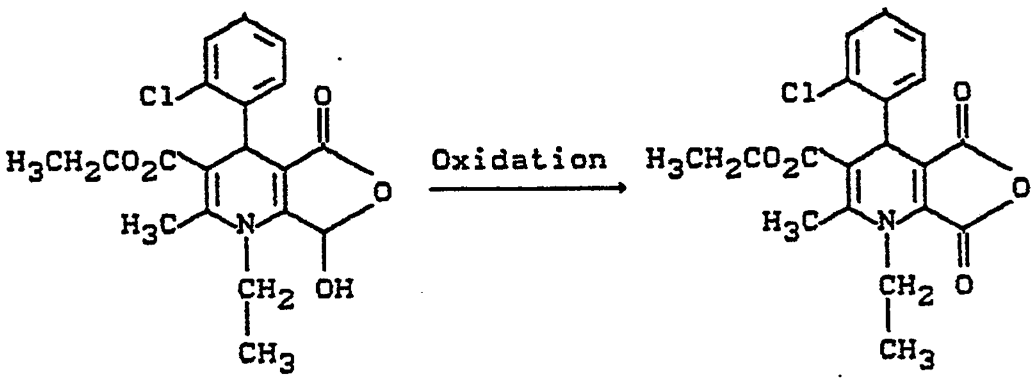


Die Oxidation erfolgt im allgemeinen mit Dimethylsulfoxid als Oxidationsmittel in Anwesenheit eines Aktivierungsmittels in geeigneten Lösemitteln. Als Aktivierungsmittel können Carbonsäureanhydride, bevorzugt Acetanhydrid oder Trifluoracetanhydrid, Carbonsäurehalogenide, bevorzugt Oxalylchlorid, oder aber Dicyclohexylcarbodiimid/Phosphorsäure, Pyridin-Schwefeltrioxid-Komplex, Phosphorpentoxid oder Chlorsulfonylisocyanat eingesetzt werden.

Als Lösemittel eignen sich die üblichen Lösemittel, die sich unter den Reaktionsbedingungen nicht verändern. Hierzu gehören bevorzugt Kohlenwasserstoffe wie Benzol, Toluol, Xylol oder Hexan, oder Ether wie Diethylether, Dioxan oder Tetrahydrofuran, oder Halogenkohlenwasserstoffe wie Methylenchlorid, Chloroform, Tetrachlorkohlenstoff, 1,2-Dichlorethan oder 1,2-Dichlorethylen, oder Gemische der genannten Lösemittel.

Besonders bevorzugt wird die Oxidation mit Dimethylsulfoxid als Oxidationsmittel in Anwesenheit von Trifluoracetanhydrid durchgeführt. Es hat sich hierbei als günstig erwiesen, Dimethylsulfoxid in großem Überschuß gleichzeitig als Lösemittel einzusetzen.

Darüberhinaus kann die Oxidation ebenso mit Oxidationsmitteln wie Chrom(VI)-Verbindungen, bevorzugt mit Chrom(VI)-oxid in verdünnter Schwefelsäure/Aceton, Essigsäure oder Pyridin, sowie Natrium- oder Kaliumdichromat, Mangandioxid oder Kaliumpermanganat durchgeführt werden, wie es in Houben-Weyl's "Methoden der organischen Chemie" Band IV/1a, 1b beschrieben wird.

Das erfindungsgemäße Verfahren wird im allgemeinen in einem Temperaturbereich von –30°C bis +60°C, bevorzugt von –10°C bis +30°C durchgeführt.

Das erfindungsgemäße Verfahren wird im allgemeinen bei Normaldruck durchgeführt, es ist aber auch möglich, das Verfahren bei Unterdruck oder bei Überdruck durchzuführen.

Das erfindungsgemäße Verfahren kann beispielsweise wie folgt durchgeführt werden:

Das Dihydropyridinlactol wird in einem Überschuß Dimethylsulfoxid gelöst und unter Kühlung mit Trifluoressigsäureanhydrid versetzt. Nach Beendigung der Reaktion wird wie üblich durch Extraktion, Chromatographie und/oder Kristallisation aufgearbeitet.

Die erfindungsgemäßen Verbindungen der allgemeinen Formel (Ib)

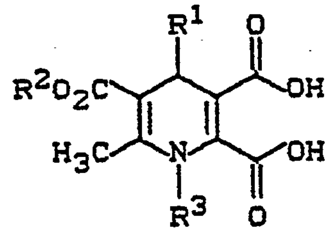

(Ib)

können hergestellt werden, indem man Dihydropyridindicarbonsäureanhydride der allgemeinen Formel (Ia) in welcher $R^1$, $R^2$ und $R^3$ die angegebene Bedeutung haben, hydrolysiert und gegebenenfalls die freien Säuren in ihre Salze überführt.

Verwendet man als Ausgangsstoff 4-(2-Chlorphenyl)-5,7-dioxo-1-ethyl-2-methyl-1,4,5,7-tetrahydrofuro[3,4-b]-pyridin-3-carbonsäureethylester, so läßt sich das Verfahren durch folgendes Schema verdeutlichen:

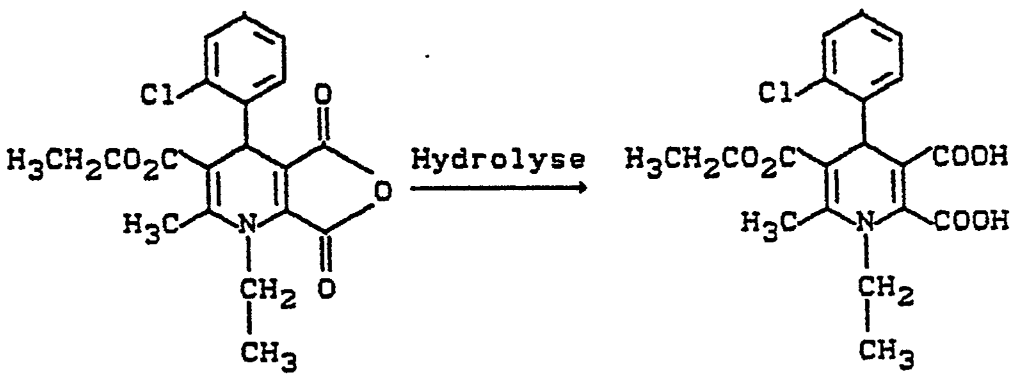


Die Hydrolyse erfolgt im allgemeinen mit Hilfe von Basen in geeigneten Lösemitteln.

Als Basen eignen sich die üblichen basischen Verbindungen. Hierzu gehören bevorzugt Alkali- oder Erdalkalihydroxide wie Natriumhydroxid, Kaliumhydroxid oder Bariumhydroxid, oder Alkalicarbonate wie Natriumcarbonat, Natriumhydrogencarbonat oder Kaliumcarbonat, oder Alkalialkoholate wie Natriummethanolat, Kaliummethanolat, Natriumethanolat, Kaliumethanolat oder Kalium-tertbutylat, oder Ammoniak, oder organische Amine wie Triethylamin oder Diisopropylamin.

Als Lösemittel eignen sich die üblichen Lösemittel, die sich unter den Reaktionsbedingungen nicht verändern. Hierzu gehören bevorzugt Wasser oder Alkohole wie Methanol, Ethanol, Propanol, Isopropanol oder Butanol, oder Ether wie Dethylether, Dioxan oder Tetrahydrofuran, oder Dimethylformamid, Hexamethylphosphorsäuretriamid, Aceton oder Acetonitril. Ebenso ist es möglich, Gemische der genannten Lösemittel zu verwenden.

Besonders bevorzugt wird die Hydrolyse mit wässrigen Alkalihydroxidlösungen wie z.B. Kalilauge oder Natronlauge in Alkoholen, wie beispielsweise Methanol, Ethanol, Propanol, Isopropanol oder Butanol als Lösemittel durchgeführt.

Die Hydrolyse wird in einem Temperaturbereich von 0°C bis +100°C, bevorzugt von +20°C bis +60°C durchgeführt.

Die Hydrolyse kann bei normalem Druck, bei Überdruck oder auch bei Unterdruck durchgeführt werden. Im allgemeinen arbeitet man bei Normaldruck.

Im allgemeinen wird die Base in eine Menge von 2 bis 6 Mol, bevorzugt von 2 bis 4 Mol, bezogen auf 1 Mol des Dihydropyridindicarbonsäureanhydrids eingesetzt.

Es hat sich hierbei als günstig erwiesen, die Base in einer Menge von mindestens 2 Mol, bezogen auf 1 Mol des Dihydropyridindicarbonsäureanhydrids einzusetzen, und in einem Schritt die entsprechenden Salze herzustellen.

Das Verfahren kann beispielsweise folgendermaßen durchgeführt werden:

Das Dihydropyridindicarbonsäureanhydrid wird in einem geeigneten Lösemittel gelöst und mit der entsprechenden Base versetzt. Die Aufarbeitung erfolgt in üblicher Art und Weise.

Die als Ausgangsstoffe eingesetzten Dihydropyridinlactole der allgemeinen Formel (II) können hergestellt werden, indem man

[A] Formylverbindungen der allgemeinen Formel (III)

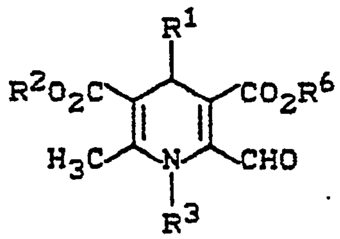

(III)

in welcher $R^1$, $R^2$ und $R^3$ die angegebene Bedeutung haben und $R^6$ für geradkettiges oder verzweigtes Alkyl mit bis zu 8 Kohlenstoffatomen steht, in geeigneten Lösemitteln, zunächst mit Base und dann mit Säure umsetzt, oder indem man
[B] Dihydropyridinlactone der allgemeinen Formel (IV)

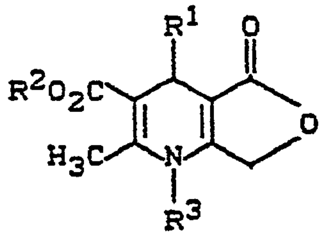

(IV)

in welcher $R^1$, $R^2$ und $R^3$ die angegebene Bedeutung haben, in geeigneten Lösemitteln, gegebenenfalls in Anwesenheit einer Base bromiert und anschließend hydrolysiert, oder direkt hydroxyliert.

Je nach Art der verwendeten Ausgangsstoffe (III), (IV) kann die Herstellung der erfindungsgemäß verwendeten Ausgangsstoffe (II) nach Verfahren A bzw. B durch folgendes Schema verdeutlicht werden:

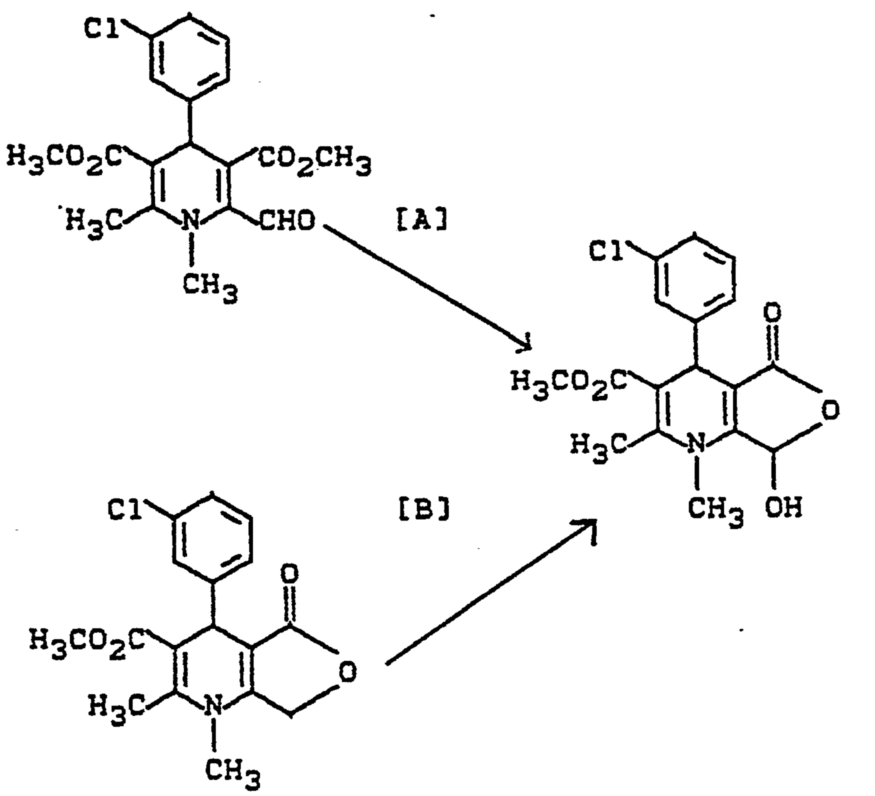

Verfahren A:

Als Lösemittel eignen sich Wasser sowie alle organischen Lösemittel, die sich unter den Reaktionsbedingungen nicht verändern. Hierzu gehören bevorzugt Alkohole wie Methanol, Ethanol, Propanol, Isopropanol oder Butanol, oder Ether wie Diethylether, Dioxan, Tetrahydrofuran, Glykolmono- oder dimethylether, oder Acetonitril, Pyridin, Dimethylformamid, Dimethylsulfoxid oder Hexamethylphosphorsäuretriamid. Ebenso ist es möglich, Gemische der genannten Lösemittel zu verwenden.

Als Basen eigenen sich die üblichen anorganischen oder organischen Basen. Hierzu gehören bevorzugt Alkalihydroxide wie Natriumhydroxid oder Kaliumhydroxid, oder Alkalialkoholate wie Natriummethylat, Natriumethylat, Kaliummethylat oder Kaliumethylat oder Kaliumtert-butylat, oder Alkalimetalle wie Natrium, oder Alkalihydride wie Natriumhydrid oder Kaliumhydrid, oder Alkaliamide wie Natriumamid oder Lithiumdiisopropylamid.

Als Säuren kommen die üblichen organischen oder anorganischen Säuren in Frage. Hierzu gehören bevorzugt Mineralsäuren wie Chlorwasserstoffsäure, Bromwasserstoffsäure, Schwefelsäure, Phosphorsäure, oder organische Carbonsäuren wie Essigsäure.

Die Durchführung erfolgt, indem man zunächst die Formylverbindungen der Formel (III) in geeigneten Lösemitteln mit 100 bis 5 Mol, bevorzugt mit 50 bis 10 Mol Base, bezogen auf 1 Mol Formylverbindung umsetzt und anschließend das Reaktionsgemisch mit Säuren behandelt. Die Aufarbeitung erfolgt in üblicher Weise.

Die Umsetzung wird im allgemeinen bei Temperaturen von 0°C bis +150°C, bevorzugt von +20°C bis +100°C durchgeführt.

Die Umsetzung kann bei normalem, aber auch bei erhöhtem oder erniedrigtem Druck erfolgen. Im allgemeinen arbeitet man bei normalem Druck.

Die als Ausgangsverbindungen eingesetzten Formylverbindungen der allgemeinen Formel (III) sind bekannt oder können nach bekannten Methoden hergestellt werden [DOS 2 629 892].

Verfahren B:

Die Bromierung wird mit den üblichen Bromierungsmitteln wie N-Bromsuccinimid oder Brom, vorzugsweise mit Brom durchgeführt.

Als Basen eignen sich hierbei die üblichen basischen Verbindungen. Hierzu gehören bevorzugte Alkalimetalle wie Natrium oder Kalium, Alkalimetallhydride wie Natriumhydrid oder Kaliumhydrid, Alkaliamide wie Natriumamid oder Lithiumdiisopropylamid, oder metallorganische Verbindungen wie Phenyllithium, n-Butyllithium, sec-Butyllithium oder tert-Butyllithium, oder Alkoholate wie Natriummethanolat, Natriumethanolat, Kaliummethanolat, Kaliumethanolat oder Kalium-tert-butanolat.

Als Lösemittel eignen sich alle organischen Lösemittel, die sich unter den Reaktionsbedingungen nicht verändern. Hierzu gehören bevorzugt Ether wie Diethylether, Dioxan oder Tetrahydrofuran, oder Kohlenwasserstoffe wie Benzol, Toluol, Xylol, Hexan, Cyclohexan, oder Erdölfraktionen. Es ist ebenso möglich, Gemische der genannten Lösemittel einzusetzen.

Die Bromierung wird in einem Temperaturbereich von –120°C bis +100°C, bevorzugt von –80°C bis +50°C durchgeführt.

Die Bromierung kann beispielsweise derart erfolgen, daß man zunächst mit 5 bis 1 Mol, bevorzugt mit 2 bis 1 Mol, besonders bevorzugt mit 1 Mol Base, bezogen auf 1 Mol der Ausgangsverbindung (IV) ein Anion erzeugt und dieses mittels Brom in das Bromid überführt. Die anschließende Überführung der Bromverbindung in die entsprechende Hydroxyverbindung der allgemeinen Formel (II) wird zweckmäßigerweise ohne Isolierung der Bromverbindung durchgeführt. Diese Hydrolyse erfolgt in an sich bekannter Weise durch Wasser, gegebenenfalls in Anwesenheit von Spuren einer Säure wie z.B. Salzsäure oder Schwefelsäure.

Das Verfahren B kann sowohl bei normalem, als auch bei erhöhtem oder erniedrigtem Druck durchgeführt werden. Im allgemeinen arbeitet man bei Normaldruck.

Die Überführung der Verbindung (IV) in die Verbindungen (II) kann jedoch auch nach anderen literaturbekannten Methoden durchgeführt werden und ist nicht auf die angegebenen Verfahren beschränkt.

Die Hydroxylierung kann ebenso mit 2-Sulfonyloxaziridin, mit Molybdänperoxid/Pyridin/Phosphit oder mit Sauerstoff/Phosphit jeweils in Anwesenheit von Basen in inerten organischen Lösemitteln durchgeführt werden wie es beispielsweise durch E. Vedejs in J. Am. Chem. Soc. 96, 5944 (1974) oder J. Org. Chem. 43, 188 (1978) oder durch J.M. Billmers, J. Finn in J. Org. Chem. 49, 3243 (1984) oder durch H.H. Wassermann, B.H. Lipschutz in Tetrahedron Letters 1975, 1731 beschrieben wird.

Die als Ausgangsverbindungen eingesetzten Lactone der allgemeinen Formel (IV) sind bekannt oder können nach bekannten Methoden hergestellt werden [DOS 3 410 645].

Die erfindungsgemäßen Verbindungen der allgemeinen Formel (I) zeigen ein wertvolles pharmakologisches Wirkspektrum.

Die blutglucosesenkende Wirkung der zu untersuchenden Substanzen wurde an männlichen Wistar-Ratten mit einem Gewicht zwischen 140 und 190 g geprüft. Die Ratten wurden zu diesem Zweck 18 h vor der Applikation der Substanzen nüchtern gesetzt. Die zu untersuchenden Substanzen wurden direkt

vor der Applikation in reinem DMSO gelöst. Die Applikation von reinem DMSO (Kontrolltiere) bzw. der in DMSO gelösten Substanzen erfolgte intravenös in die Schwanzvene der Ratten.

Die Blutentnahme erfolgte bei jeder Ratte 30, 60 und 120 min nach der Applikation aus dem retroorbitalen Venenplexus. Jeweils 30 µl Blut wurden mit einem automatischen Dilutor entnommen und mit 0,3 ml Uranylacetat (0,16%) enteiweißt. Nach der Zentrifugation wurde die Glucose im Überstand nach der Glucoseoxidase-Methode mit 4-Amino-phenazon als Farbreagenz an einem Gemsaec Fastanalyzer photometrisch bestimmt. Die Auswertung der Ergebnisse erfolgte mit dem t-Test nach Student, als Signifikanzgrenze wurde $p < 0,05$ gewählt.

Substanzen, die bei Ratten zu einem Zeitpunkt eine signifikante Reduktion der Blutglucosekonzentration um mindestens 10% bewirkten, verglichen mit der Kontrollgruppe, die nur DMSO intravenös erhielt, wurden als wirksam angegeben.

Die nachfolgende Tabelle 1 enthält die gefundenen Veränderungen der Blutglucosekonzentrationen in Prozent der Kontrolle.

Tabelle 1

| Substanz (Beispiel Nr.) | Abnahme der Blutglucosekonzentration in % der Kontrolle 1 mg/kg KG i.v. |
|---|---|
| 2 | 22 |
| 3 | 23 |

Zur vorliegenden Erfindung gehören pharmazeutische Zubereitungen, die neben nichttoxischen, inerten pharmazeutisch geeigneten Trägerstoffen einen oder mehrere erfindungsgemäße Verbindungen enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tragesdosis entspricht.

Unter nichttoxischen, inerten pharmazeutischen geeigneten Trägerstoffen sind feste, halbfeste oder flüssige Verdünnungsmittel, Füllstoffe oder Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärke, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gewichtsprozent der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch das Mischen des oder der Wirkstoffe mit dem oder den Trägerstoffen.

Zur vorliegenden Erfindung gehört auch die Verwendung der Verbindungen der allgemeinen Formel (i) und/oder deren Salze, sowie deren pharmazeutischen Zubereitungen, die die Verbindungen der Formel (I) und/oder deren Salze enthalten, in der Human- und Veterinärmedizin, zur Verhütung, Verbesserung und/oder Heilung von Erkrankungen des Kohlenhydratstoffwechsels.

Im allgemienen hat es sich sowohl in der Human- als auch in der Veterinärmedizin als vorteilhaft erwiesen, den oder die erfindungsgemäßen Wirkstoffe in Gesamtmengen von etwa 0,5 bis 500, vorzugsweise 50 bis 100 mg/kg Körpergewicht je 24 Stunden, gegebenenfalls in Form mehrerer Einzeldosen, zur Erzielung der gewünschten Ergebnisse zu verabreichen. Eine Einzelgabe enthält den oder die erfindungsgemäßen Wirkstoffe, vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objektes, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Herstellungsbeispiele

I. Ausgangsprodukt

Beispiel 1

4-(2-Chlorphenyl)-1-ethyl-7-hydroxy-2-methyl-5-oxo-1,4,5,7-tetrahydrofuro[3,4-b]pyridin-3-carbonsäureisopropylester

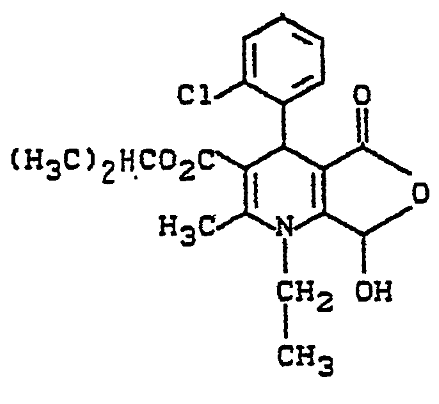

Verfahren A.

5 mmol 4-(2-Chlorphenyl)-1-ethyl-2-formyl-6-methyl-1,4-dihydropyridin-3,5-dicarbonsäure-3-methylester-5-isopropylester werden in 40 mmol 2 N KOH vorgelegt und im Wasserbad kurz auf 40°C erwärmt. Danach läßt man eine Stunde bei Raumtemeperatur nachrühren. Die Lösung wird mit Aktivkohle geklärt und mit Salzsäure angesäuert, der Niederschlag wird abgesaugt.
Ausbeute: 30% der Theorie
FP: 145–147°C

Verfahren B:

60 mmol Diisopropylamin werden in 100 ml Tetrahydrofuran vorgelegt. Bei einer Temperatur von 0°C werden unter Stickstoffstrom 50 mmol Butyllithium zugegeben. Danach wird auf −78°C gekühlt und eine Lösung aus 50 ml 4-(2-Chlorphenyl)-1-ethyl-2-methyl-5-oxo-1,4,5,7-tetrahydrofuro[3,4-b]pyridin-3-carbonsäure-isopropylester (in Tetrahydrofuran gelöst) zugetropft. Es wird 15 Minuten bei −78°C gerührt und mit Hilfe von Stickstoff wird diese Lösung in einer Lösung von 50 mmol $Br_2$ und 50 ml Tetrahydrofuran gepumpt, sofort danach gibt man 50 mmol Cyclohexen dazu und läßt auf Raumtemperatur erwärmen, engt ein, löst in Dimethylsulfoxid und gibt bis zur beginnenden Trübung Wasser hinzu. Man läßt 2 Stunden stehen, fällt mit Wasser aus, saugt ab und trennt mit $CHCl_3$/MeOH 9:1 an Kieselgel.
Ausbeute: 30% der Theorie
FP: 145–147°C

II. Endprodukt

Beispiel 2

4-(2-Chlorphenyl)-5,7-dioxo-1-ethyl-2-methyl-1,4,5,7-tetrahydrofuro[3,4-b]pyridin-3-carbonsäure-isopropylester

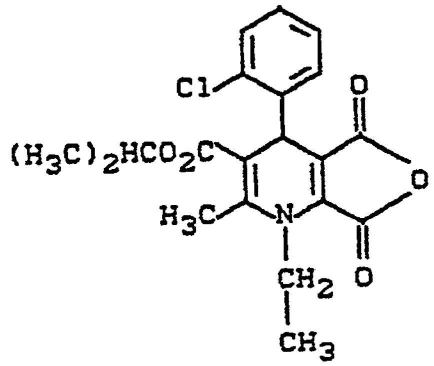

3,9 g (10 mmol) 4-(2-Chlorphenyl)-1-ethyl-7-hydroxy-2-methyl-5-oxo-1,4,5,7-tetrahydrofuro[3,4-b]pyridin-3-carbonsäure-isopropylester werden in 6 ml absolutem Dimethylsulfoxid gelöst und unter Kühlung mit 3 ml Trifluoressigsäureanhydrid versetzt und 1 Stunde bei Raumtemperatur gerührt. Es wird rasch an Kieselgel chromatographiert (Toluol:Essigester = 8:2) und der gelbe Fleck isoliert. Nach dem Einengen wird mit wenig Methanol kristallisiert, sofort abgesaugt und getrocknet.
Ausbeute: 1,65 g (42,3% der Theorie)
FP: 87–90°C

Beispiel 3

4-(2-Chlorphenyl)-1,4-dihydro-1-ethyl-2-methyl-pyridin-3,5,6-tricarbonsäure-3-isopropylester Dinatriumsalz

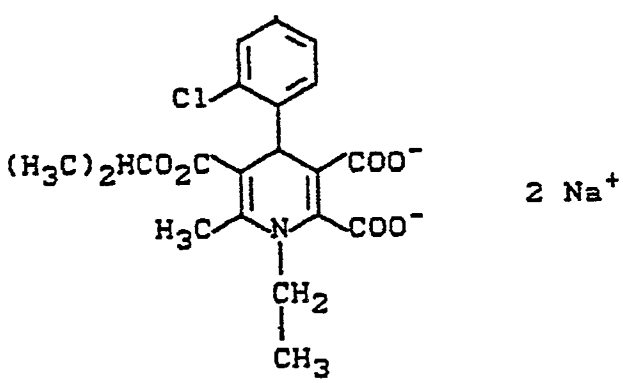

120 mg 4-(2-Chlorphenyl)-5,7-dioxo-1-ethyl-2-methyl-1,4,5,7-tetrahydrofuro[3,4-b]pyridin-3-carbonsäure-isopropylester werden in 20 ml tert-Butanol in der Wärme gelöst und sofort solange mit 2 Equivalenten 0,2 N wässriger Natronlauge versetzt. Es wird eingefroren und gefriergetrocknet.
Ausbeute: 120 mg
FP: amorph.
Analog Beispiel 2 werden hergestellt:

Beispiel 4:

4-(2-Chlorphenyl)-5,7-dioxo-1-propyl-2-methyl-1,4,5,7-tetrahydrofuro[3,4-b]pyridin-3-carbonsäure-isopropylester (Ausbeute 55%).

FP: 111°C

1H-NMR ($CD_2Cl_2$) δ=0.85 (d,3H), 1.0 (t,3H), 1.1 (d,3H), 1.7 (m,2H), 2.5 (s,3H), 4.0 (m,2H), 4.8 (m,1H), 5.5 (s,1H), 7.1–7.4 (m,4H) ppm.

Beispiel 5:

4-(3-Chlorphenyl)-5,7-dioxo-1-ethyl-2-methyl-1,4,5,7-tetrahydrofuro[3,4-b]pyridin-3-carbonsäure-isopropylester (Ausbeute 44%).

FP: 106°C

1H-NMR ($CD_2Cl_2$) δ=0.95 (d,3H), 1.2 (d,3H), 1.3 (t,3H), 2.5 (s,3H), 4.0–4.3 (m,2H), 4.9 (qq,1H), 5.3 (s,1H), 7.1–7.3 (m,4H) ppm.

Beispiel 6

4-(2-Chlorphenyl)-1,2-dimethyl-5,7-dioxo-1,4,5,7-tetrahydrofuro-[3,4-b]pyridin-3-carbonsäure-isopropylester

FP: amorph (Ausbeute 46%)

1H-NMR ($CD_2Cl_2$) δ=0.85 (d,3H), 1.2 (d,3H), 2.5 (s,3H), 3.6 (s,3H), 4.85 (hept.,1H), 5.5 (s,1H), 7.1–7.4 (m,4H) ppm.

Beispiel 7

4-(3-Chlor-2-thienyl)-1-ethyl-2-methyl-5,7-dioxo-1,4,5,7-tetrahydrofuro[3,4-b]pyridin-3-carbonsäure-isopropylester

FP: 133°C (Ausbeute 62%)

1H-NMR ($CDCl_3$) δ=1.0 (d,3H), 1.25 (d,3H), 1.3 (t,3H), 2.5 (s,3H), 4.05 (qd,1H), 4.3 (qd,1H), 5.0 (hept.,1H), 5.4 (s,1H), 6.85 (d,1H), 7.2 (d,1H) ppm.

Beispiel 8

4-(2-Chlorphenyl)-1-ethyl-2-methyl-5,7-dioxo-1,4,5,7-tetrahydrofuro[3,4-b]pyridin-3-carbonsäure-isobutylester.

FP: amorph (Ausbeute 48%)

1H-NMR ($CD_2Cl_2$) δ=0.7 (d,3H), 0.8 (d,3H), 1.3 (t,3H), 1.8 (m,1H), 2.5 (s,3H), 3.7 (dd,1H), 4.1 (qd,1H), 4.2 (qd,1H), 5.5 (s,1H), 7.1–7.4 (m,4H) ppm.

Beispiel 9

1-Ethyl-2-methyl-4-(2-methylphenyl)-5,7-dioxo-1,4,5,7-tetrahydrofuro[3,4-b]pyridin-3-carbonsäure-isopropylester.

FP: 117–118°C (Ausbeute 65%)

1H-NMR ($CDCl_3$) δ=0.8 (d,3H), 1.2 (d,3H), 1.35 (t,3H), 2.5 (s,3H), 2.6 (s,3H), 4.05 (qd,1H), 4.25 (qd,1H), 4.9 (hept.,1H), 5.2 (s,1H) ppm.

Beispiel 10:

4-(2,4-Dichlorphenyl)-1-ethyl-2-methyl-pyridin-3,5,6-tricarbonsäure-3-isopropylester-Dinatrium-salz (Ausbeute 85%).

1H-NMR ($D_2O$) δ=1.1 (d,3H, 1.2 (t,3H), 1.2 (d,3H), 2.25 (s,3H), 3.5 (qd,1H), 3.7 (qd,1H), 4.9 (hept.,1H), 5.2 (s,1H), 7.2–7.4 (m,4H) ppm.

**Patentansprüche**

1. Dihydropyridinverbindungen der allgemeinen Formel I

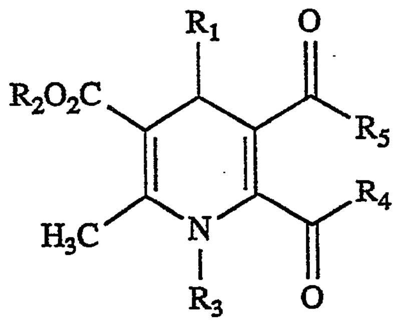

(I)

in welcher
$R_1$ für Phenyl oder Thienyl steht, wobei die genannten Reste bis zu 2 gleiche oder verschiedene Substituenten der Reihe, Fluor, Chlor, Alkyl, Alkoxyl mit jeweils bis zu 3 Kohlenstoffatomen, Trifluormethyl, Nitro oder Cyano tragen können,
$R_2$ für geradkettiges oder verzweigtes Alkyl mit bis zu 6 Kohlenstoffatomen steht, das durch ein Sauerstoffatom in der Alkylkette unterbrochen sein kann,
$R_3$ für geradkettiges oder verzweigtes Alkyl mit bis zu 6 Kohlenstoffatomen steht, das durch Hydroxy substituiert sein kann und
$R_4$ und $R_5$ jeweils für Hydroxy stehen, oder
$R_4$ und $R_5$ zusammen für -O- stehen,
in Form ihrer Isomeren, Isomerengemische, optischen Antipoden oder Racemate sowie ihrer physiologisch unbedenklichen Salze.

2. Verbindungen der allgemeinen Formel (I) gemäß Anspruch 1, in welcher
$R_1$ für Phenyl steht, das bis zu 2-fach gleich oder verschieden substituiert sein kann durch Chlor, Alkyl mit bis zu 3 Kohlenstoffatomen, Trifluormethyl oder Nitro,
$R_2$ für geradkettiges oder verzweigtes Alkyl mit bis zu 4 Kohlenstoffatomen steht, das in der Alkylkette durch 1 Sauerstoffatom unterbrochen sein kann,
$R_3$ für geradkettiges oder verzweigtes Alkyl mit bis zu 4 Kohlenstoffatomen steht und
$R_4$ und $R_5$ jeweils für Hydroxy stehen, oder
$R_4$ und $R_5$ zusammen für -O- stehen,
in Form ihrer Isomeren, Isomerengemische, optischen Antipoden oder Racemate sowie ihrer physiologisch unbedenklichen Salze.

3. Verbindungen der allgemeinen Formel (I) gemäß Ansprüchen 1 bis 2 zur Bekämpfung von Erkrankungen.

4. Verfahren zur Herstellung von Verbindungen der allgemeinen Formel (I)

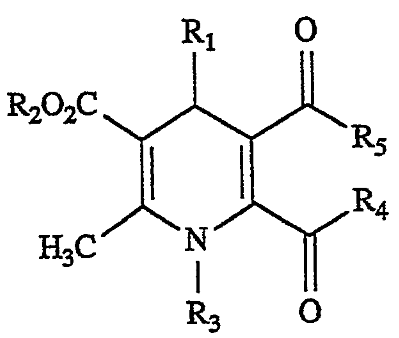

(I)

in welcher

$R_1$ für Phenyl oder Thienyl steht, wobei die genannten Rste bis zu 2 gleiche oder verschiedene Substituenten der Reihe, Fluor, Chlor, Alkyl, Alkoxy mit jeweils bis zu 3 Kohlenstoffatomen, Trifluormethyl, Nitro oder Cyano tragen können,
$R_2$ für geradkettiges oder verzweigtes Alkyl mit bis zu 6 Kohlenstoffatomen steht, das durch ein Sauerstoffatom in der Alkylkette unterbrochen sein kann,
$R_3$ für geradkettiges oder verzweigtes Alkyl mit bis zu 6 Kohlenstoffatomen steht, das duch Hydroxy substituiert sein kann und
$R_4$ und $R_5$ jeweils für Hydroxy stehen, oder
$R_4$ und $R_5$ zusammen für -O- stehen, dadurch gekennzeichnet, daß man zur Herstellung solcher Verbindungen der allgemeinen Formel (I), in welcher

$R_4$ und $R_5$ zusammen für -O- stehen,
Dihydropyridinlactole der allgemeinen Formel (II)

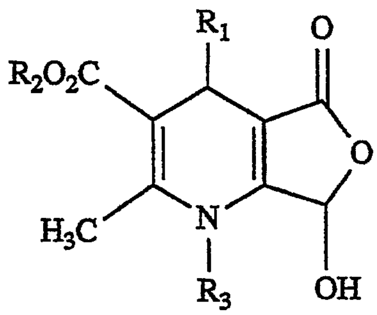
(II)

in welcher
$R_1$, $R_2$ und $R_3$ die oben angegebene Bedeutung haben,
in inerten Lösemitteln, in Ggenwart von Oxidationsmittel bei Temperaturen zwischen –30°C und +60°C oxidiert und die erhaltenen Anhydride gegebenenfalls in Gegenwart von Basen in inerten Lösemitteln bei Temperaturen zwischen 0 bis 100°C hydrolysiert zu Verbindungen der allgemeinen Formel (I), in welcher $R_4$ und $R_5$ jeweils für Hydroxy stehen.

5. Verfahren gemäß Anspruch 4, dadurch gekennzeichnet, daß man als Oxidationsmittel Dimethylsulfoxid, Chrom (VI)-Verbindungen, Natrium- oder Kaliumdichromat, Mangandioxid oder Kaliumpermanganat verwendet.

6. Arzneimittel enthaltend mindestens eine Verbindung der allgemeinen Formel (I) gemäß Ansprüchen 1 bis 2.

7. Verfahren zur Herstellung von Arzneimitteln, dadurch gekennzeichnet, daß man Verbindungen der allgemeinen Formel (I) gemäß Ansprüchen 1 bis 2, gegebenenfalls unter Verwendung von Hilfs- und Trägerstoffen in eine geeignete Applikationsform überführt.

8. Verwendung von Verbindungen der allgemeinen Formel (I) gemäß Ansprüchen 1 bis 2 bei der Herstellung von Blutzucker-beeinflussenden Arzneimitteln.

## Claims

1. Dihydropyridine compounds of the general formula (I)

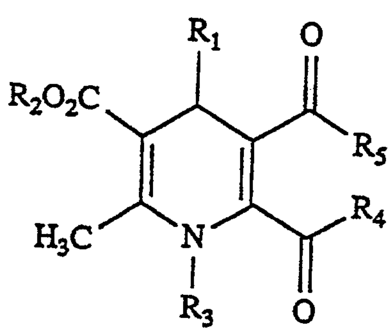
(I)

in which
$R_1$ represents phenyl or thienyl, it being possible for the radicals mentioned to carry up to 2 identical or different substituents from the series comprising fluorine, chlorine, alkyl, alkoxy with in each case up to 3 carbon atoms, trifluoromethyl, nitro and cyano,

$R_2$ represents straight-chain or branched alkyl which has up to 6 carbon atoms and can be interrupted by an oxygen atom in the alkyl chain,
$R_3$ represents straight-chain or branched alkyl which has up to 6 carbon atoms and can be substituted by hydroxyl and
$R_4$ and $R_5$ each represent hydroxyl, or
$R_4$ and $R_5$ together represent -O-,
in the form of their isomers, isomer mixtures, optical antipodes or racemates, and their physiologically acceptable salts.

2. Compounds of the general formula (I) according to Claim 1, in which
$R_1$ represents phenyl, which can be substituted by up to 2 identical or different substituents from the group comprising chlorine, alkyl with up to 3 carbon atoms, trifluoromethyl and nitro,
$R_2$ represents straight-chain or branched alkyl which has up to 4 carbon atoms and can be interrupted in the alkyl chain by 1 oxygen atom,
$R_3$ represents straight-chain or branched alkyl with up to 4 carbon atoms and
$R_4$ and $R_5$ each represent hydroxyl, or
$R_4$ and $R_5$ together represent -O-,
in the form of their isomers, isomer mixtures, optical antipodes or racemates and their physiologically acceptable salts.

3. Compounds of the general formula (I) according to Claims 1 and 2 for combating diseases.

4. Process for the preparation of compounds of the general formula (I)

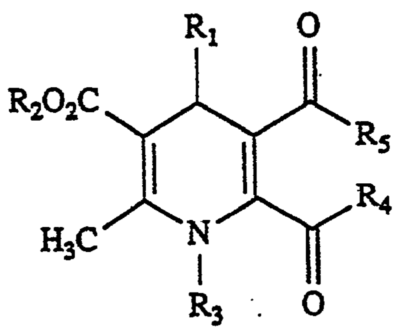

(I)

in which
$R_1$ represents phenyl or thienyl, it being possible for the radicals mentioned to carry up to 2 identical or different substituents from the series comprising fluorine, chlorine, alkyl, alkoxy with in each case up to 3 carbon atoms, trifluoromethyl, nitro and cyano,
$R_2$ represents straight-chain or branched alkyl which has up to 6 carbon atoms and can be interrupted by an oxygen atom in the alkyl chain,
$R_3$ represents straight-chain or branched alkyl which has up to 6 carbon atoms and can be substituted by hydroxyl and
$R_4$ and $R_5$ each represent hydroxyl, or
$R_4$ and $R_5$ together represent -O-, characteriezed in that, to prepare those compounds of the general formula (I)
in which
$R_4$ and $R_5$ together represent -O-, dihydropyridinelactols of the general formula (II)

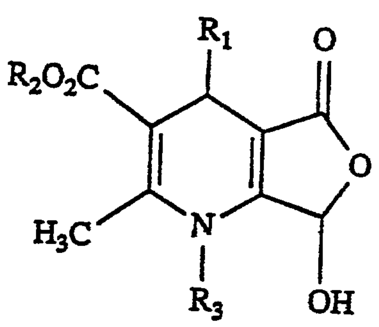

(II)

in which
$R_1$, $R_2$ and $R_3$ have the abovementioned meaning, are oxidized in inert solvents in the presence of oxidizing agents at temperatures between –30°C and +60°C and the resulting anhydrides are hydrolysed, if appropriate in the presence of bases, in inert solvents at temperatures between 0 and 100°C, to give

compounds of the general formula (I)
in which
$R_4$ and $R_5$ each represent hydroxyl.

5. Process according to Claim 4, characterized in that dimethyl sulphoxide, chromium (VI) compounds, sodium dichromate or potassium dichromate, manganese dioxide or potassium permanganate are used as the oxidizing agent.

6. Medicaments containing at least one compound of the general formula (I) according to Claims 1 and 2.

7. Process for the preparation of medicaments, characterized in that compounds of the general formula (I) according to Claims 1 and 2 are converted into a suitable administration form, if appropriate using auxiliaries and excipients.

8. Use of compounds of the general formula (I) according to Claims 1 and 2 in the preparation of medicaments which influence blood sugar.

## Revendications

1. Dérivés de dihydropyridines répondant à la formule générale I

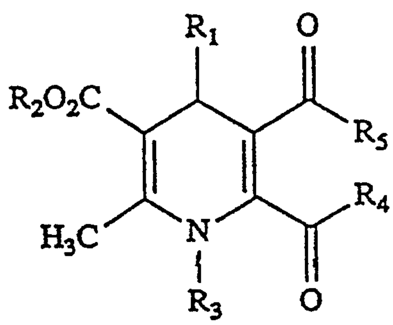

(I)

dans laquelle
$R_1$ représente un groupe phényle ou thiényle, ces groupes pouvant porter jusqu'à deux substituants identiques ou différents choisis parmi le fluor, le chlore, les groupes alkyle, alcoxy contenant chacun jusqu'à 3 atomes de carbone, trifluorométhyle, nitro ou cyano,
$R_2$ représente un groupe alkyle à chaîne droite ou ramifiée contenant jusqu'à 6 atomes de carbone et qui peut être interrompu dans la chaîne alkyle par un atome d'oxygène,
$R_3$ représente un groupe alkyle à chaîne droite ou ramifiée contenant jusqu'à 6 atomes de carbone et qui peut être substitué par un groupe hydroxy et
$R_4$ et $R_5$ représentent chacun un groupe hydroxy, ou bien
$R_4$ et $R_5$ représentent ensemble -O-,
sous la forme de leurs isoméres, de mélanges d'isomères, d'antipodes optiques ou de racémates, ainsi que leurs sels acceptables pour l'usage pharmaceutique.

2. Composés de formule générale I selon la revendication 1, dans laquelle
$R_1$ représente un groupe phényle qui peut porter jusqu'à deux substituants identiques ou différents choisis parmi le chlore, les groupes alkyle contenant jusqu'à 3 atomes de carbone, trifluorométhyle ou nitro
$R_2$ représente un groupe alkyle à chaîne droite un ramifiée contenant jusqu'à 4 atomes de carbone et qui peut être interrompu dans la chaîne alkyle par un atome d'oxygène,
$R_3$ représente un groupe alkyle à chaîne droite ou ramifiée contenant jusqu'à 4 atomes de carbone et
$R_4$ et $R_5$ représentent chacun un groupe hydroxy, ou bien
$R_4$ et $R_5$ représentent ensemble -O-,
sous la forme de leurs isomères, des mélanges d'isomères, des antipodes optiques ou des racémates, ainsi que leurs sels acceptables pour l'usage pharmaceutique.

3. Composés de formule générale I selon les revendications 1 à 2, pour le traitement de maladies.

4. Procédé de préparation des composés de formule générale I

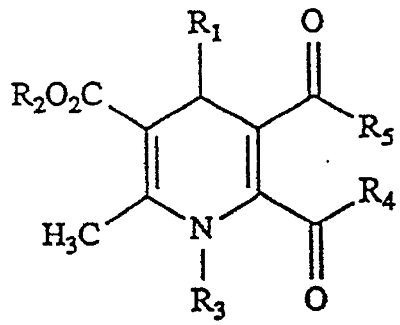

(I)

dans laquelle

$R_1$ représente un groupe phényle ou thiényle, ces groupes pouvant porter jusqu'à deux substituants identiques ou différents choisis parmi le fluor, le chlore, les groupes alkyle, alcoxy contenant chacun jusqu'à 3 atomes de carbone, trifluorométhyle, nitro ou cyano,

$R_2$ représente un groupe alkyle à chaîne droite ou ramifiée contenant jusqu'à 6 atomes de carbone et qui peut être interrompu par un atome d'oxygène dans la chaîne alkyle,

$R_3$ représente un groupe alkyle à chaîne droite ou ramifiée contenant jusqu'à 6 atomes de carbone et qui peut être substitué par un groupe hydroxy, et

$R_4$ et $R_5$ représentent chacun un groupe hydroxy, ou bien

$R_4$ et $R_5$ représentent ensemble -O-, caractérisé en ce que, pour préparer les composés de formule générale I dans laquelle

$R_4$ et $R_5$ représentent ensemble -O-, on oxyde des dihydropyridine-lactols de formule générale II

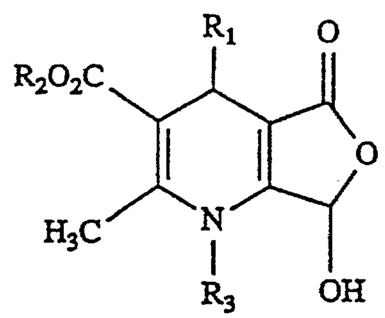

(II)

dans laquelle

$R_1$, $R_2$ et $R_3$ ont les significations indiquées ci-dessus, dans des solvants inertes, en présence d'agents oxydants, à des températures allant de –30 à +60°C, et le cas échéant on hydrolyse les anhydrides ainsi obtenus en présence de bases dans des solvants inertes à des températures de 0 à 100°C, ce qui donne des composés de formule générale I dans laquelle

$R_4$ et $R_5$ représentent chacun un groupe hydroxy.

5. Procédé selon la revendication 4, caractérisé en ce que l'on utilise en tant qu'agents oxydants le diméthylsulfoxyde, des composés du chrome-VI, le bichromate de sodium ou de potassium, le bioxyde de manganèse ou le permanganate de potassium.

6. Médicament contenant au moins un composé de formule générale I selon les revendication 1 à 2.

7. Procédé de préparation de médicaments, caractérisé en ce que l'on met les composés de formule générale I selon les revendications 1 à 2, le cas échéant avec utilisation de produits auxiliaires et véhicules, sous une forme d'administration appropriée.

8. Utilisation des composés de formule générale I selon les revendications 1 à 2 pour la préparation de médicaments agissant sur la teneur en sucre du sang.